# EUROPEAN PATENT SPECIFICATION

(11) **EP 4 161 408 B1**
(45) Date of publication and mention of the grant of the patent: **23.10.2024**
(21) Application number: 20731820.5
(22) Date of filing: 04.06.2020
(51) Int. Cl.: A61B 17/12

(54) **FLOW RESTRICTOR FOR AN EMBOLIZATION DEVICE**
DURCHFLUSSBEGRENZER FÜR EINE EMBOLISIERUNGSVORRICHTUNG
LIMITEUR DE DÉBIT POUR UN DISPOSITIF D'EMBOLISATION

(43) Date of publication of application: 12.04.2023
(73) Proprietor: Clearstream Technologies Limited, Enniscorthy, County Wexford (IE)
(72) Inventor: WALL, Sean, Enniscorthy, County Wexford (IE); O´SHEA, John, Enniscorthy, County Wexford (IE); MCDONAGH, Donal, Enniscorthy, County Wexford (IE)
(74) Representative: Hoffmann Eitle
(86) International application number: PCT/EP2020/065451
(87) International publication number: WO 2021/244744

(56) References cited:
- EP-A1- 3 632 350
- US-A1- 2009 062 838
- US-A1- 2015 112 376
- US-A1- 2016 030 047
- US-A1- 2016 206 319
- US-A1- 2018 338 824
- US-A1- 2020 078 022

## Description

### Field

The present disclosure relates to a flow restrictor for an embolization device for promoting clot formation in a bodily lumen. The present disclosure also relates to an embolization device and a method of manufacturing an embolization device.

### Background

Embolisation devices may be deployed in the vasculature at a particular location by a medical practitioner so as to promote blood clot formation and ultimately occlude the blood vessel. Typically, an embolisation device is pushed from a loading tube and through a guide (delivery) catheter in a distal direction using a delivery wire until a point of deployment within a bodily lumen is reached. Once the device reaches the required point of deployment, the device is deployed from the guide catheter.

The embolization devices may include a flow restrictor having an occlusion membrane which is configured to mechanically inhibit blood flow through the blood vessel when the embolization device is deployed, in order to assist in embolizing the vessel and facilitate thrombosis at the implantation site.

Certain properties may be desired for an occlusion membrane.

On the one hand, certain properties are preferred of the membrane during delivery and deployment. For example, it may be desirable for the occlusion membrane to have sufficient mechanical strength to be able to restrict bloodflow in the vessel. It may also be desirable that the surface of the membrane have sufficient lubricity such that it is able to be pushed through a loading tube and a delivery catheter in a collapsed configuration. If the lubricity is not sufficient, the membrane may tear or be damaged during delivery due to friction between the membrane and the tube or catheter.

On the other hand, other properties of the membrane are preferred to reduce the risk of damage to the membrane during manufacture of the embolisation device. For example, materials with high elasticity may be preferred as they are less likely to tear during the assembly process, which may involve manipulating and stretching the membrane to mount the membrane on the device.

Materials which have good properties for delivery and deployment (e.g. are good flow restrictors with sufficient lubricity) tend to be prone to tearing during assembly of the embolisation device. On the other hand, materials with a sufficient elasticity to avoid tearing during assembly tend to be sensitive to the environmental factors experienced by the membrane during use, such as temperature and humidity, and exhibit a worse lubricity which affects the ability of the embolisation device to be delivered through a delivery catheter. This is especially true at the membrane thicknesses used in embolization devices, for example thin film membranes (e.g. membranes with a thickness of 100 micrometres or less).

Accordingly, there is a need for occlusion membranes with preferable properties both during assembly and during delivery and deployment.

US 2009/062838 A1 discloses an occlusion device for occluding a body vessel. The occlusion device includes a first hub extending from a proximal end to a distal end and a tubular wall defining a lumen having a central axis. A plurality of arcuate legs are attached to the first hub and extend distally to a distal portion. The legs extend radially away from the central axis in an open configuration and extend substantially along the central axis in a closed configuration. A biocompatible material is attached to the plurality of legs to form an occlusive barrier when deployed within the body vessel. The biocompatible material either extends along and between each of the plurality of legs or it forms a disk attached to at least one leg.

### Summary

According to an embodiment of the invention and as defined in claim 1, there is provided a flow restrictor for an embolization device, the flow restrictor having an inner hole for receiving a stem of the embolization device to mount the flow restrictor on the embolization device, wherein the flow restrictor comprises: a membrane for restricting flow in the bodily lumen, having a first elasticity; and a reinforcing section having a second elasticity and at least partially surrounding the inner hole. Advantageously, the flow restrictor comprising the reinforcing section about the inner hole inhibits tearing during use (for instance during mounting of the flow restrictor on the stem).

The reinforcing section may entirely surround the perimeter of the inner hole, and preferably the reinforcing section may define the entire perimeter of the inner hole.

The reinforcing section may be annular in shape, and the inner radial surface of the annulus may define the inner hole.

The membrane may be annular in shape. The membrane may also be cup shaped.

The reinforcing section and the membrane may abut each other.

The membrane may define at least a part of the outermost perimeter of the flow restrictor, and may define the outermost perimeter of the flow restrictor.

The average diameter of the reinforcing section may be less than 40% of the average diameter of the membrane. This ensures that the lubricity of the flow restrictor when being delivered through a delivery catheter is mainly determined by the surface properties of the membrane (i.e. the outermost parts of the flow restrictor which abut the inner surface of the delivery catheter), improving the deliverability of the flow restrictor.

The average diameter of the reinforcing section may be greater than 5% of the average diameter of the membrane. Diameters in this range have shown to provide a good amount of resistance to tearing of the flow restrictor at the inner hole.

The second elasticity may be greater than the first elasticity. Advantageously, the greater elasticity assists the inner hole in being mounted onto a stem without tearing and to return to its original diameter for a more secure fit on the stem. The reinforcing section may comprise or consist of a polyurethane such as silicone polycarbonate urethane or polycarbonate urethane.

The second elasticity may be lower than the first elasticity. This provides rigidity to the inner hole and lowers the risk of tearing during assembly. The reinforcing section may comprise or consist of cyanoacrylate.

The reinforcing section may comprise or consist of a thermoplastic material.

The membrane may comprise or consist of: ultra-high-molecular-weight polyethylene; polytetrafluoroethylene, such as an expanded polytetrafluoroethylene or an electrospun polytetrafluoroethylene; a polytetrafluoroethylene composite; silicone polycarbonate urethane; or polycarbonate urethane.

The reinforcing section may comprise or consist of a fibrous material.

The membrane may comprise or consist of a fibrous material.

According to this embodiment of the invention and as defined in claim 1, the reinforcing section is at least partially interspersed with the membrane. This provides additional adherence between the reinforcing section and the membrane, further reducing the risk of tearing. For example, the membrane may be made of a fibrous material, and the reinforcing section may be interspersed between the fibers of the membrane material.

The reinforcing section may be a fibrous material.

According to a second aspect, there is provided an embolization device for promoting clot formation in a bodily lumen, the embolization device adapted to have a collapsed delivery configuration and an expanded deployed configuration, the embolization device comprising: a stem; and a flow restrictor according to the first aspect and being disposed on the stem such that the stem passes through the inner hole.

According to a third aspect there is provided a method of manufacturing an embolization device, the method comprising: providing a stem; providing a flow restrictor, the flow restrictor having an inner hole therein, the flow restrictor comprising: a membrane for restricting flow in the bodily lumen, having a first elasticity; and a reinforcing section having a second elasticity and at least partially surrounding the inner hole; and disposing the flow restrictor on the stem such that the stem passes through the inner hole.

Disposing the flow restrictor on the stem may comprise stretching the inner hole in at least one direction, thereafter passing the stem therethrough and thereafter releasing the inner hole to form a frictional fit between the inner hole and the stem.

Disposing the flow restrictor on the stem may comprise forming an interference fit between the flow restrictor and the stem. Alternatively or additionally, disposing the flow restrictor on the stem may comprise bonding the flow restrictor to the stem by welding or adhesive. Alternatively or additionally, disposing the flow restrictor on the stem may comprise disposing the flow restrictor longitudinally in between a first and second holding rings, the first and second holding rings attached to the stem by an interference fit, welding or adhesive.

The reinforcing section may comprise or consist of a thermoplastic material.

Disposing the flow restrictor on the stem may comprise heating the thermoplastic material of the reinforcing section before passing the stem through the inner hole.

According to another embodiment of the invention and as defined in claim 11, there is provided a method of manufacturing a flow restrictor for an embolization device, the method comprising: providing a membrane for restricting flow in a bodily lumen, having a first elasticity; providing a reinforcing section having a second elasticity; and providing an inner hole extending through the flow restrictor, wherein the reinforcing section at least partially surrounds the inner hole. The reinforcing section is at least partially interspersed with the membrane.

Providing the membrane of the flow restrictor may comprise providing a layer of first material.

Providing the reinforcing section may comprise adding a second material to the first material in and/or on a portion of the first material.

The inner hole may be formed by forming a hole through a combination of the first material and the second material.

The inner hole may be formed in the layer of first material before adding the second material.

The first material may be a fibrous material.

Providing the reinforcing section may comprise adding a second material to the fibrous first material when the second material is in a liquid phase. This increases adherence between the first and second materials.

The second material may be a fibrous material.

### Brief description of the drawings

To enable better understanding of the present disclosure, and to show how the same may be carried into effect, reference will now be made, by way of example only, to the accompanying schematic drawings, in which:
FIG. 1 shows a side view of an embolization device in an unconstrained configuration according to one or more embodiments shown and described herein;
FIG. 2 shows a side view of the embolization device of FIG. 1 in a collapsed delivery configuration within a delivery catheter according to one or more embodiments shown and described herein;
FIG. 3 shows a side view of the embolization device of FIG. 3 in an expanded deployed configuration in a bodily lumen according to one or more embodiments shown and described herein;
FIG. 4A illustrates a first step an exemplary process for mounting a flow restrictor onto a stem of an embolization device according to one or more embodiments shown and described herein;
FIG. 4B illustrates a second step of an exemplary process for mounting a flow restrictor onto a stem of an embolization device according to one or more embodiments shown and described herein;
FIG. 4C illustrates a third step of an exemplary process for mounting a flow restrictor onto a stem of an embolization device according to one or more embodiments shown and described herein;
FIG. 4D illustrates a fourth step of an exemplary process for mounting a flow restrictor onto a stem of an embolization device according to one or more embodiments shown and described herein;
FIG. 5A shows a front view of a flow restrictor according to one or more embodiments shown and described herein;
FIG. 5B shows a side view of a flow restrictor according to one or more examples not falling within the scope of the appended claims;
FIG. 5C shows a side view of a flow restrictor according to one or more examples not falling within the scope of the appended claims;
FIG. 5D shows a side view of a flow restrictor according to one or more examples not falling within the scope of the appended claims;
Fig. 5E shows a flow restrictor according to one or more embodiments;
FIG. 6A shows an articulating joint of a device according to one or more embodiments shown and described herein;
FIG. 6B shows a flexible joint of a device according to one or more embodiments shown and described herein;
FIG 6C shows an inflexible joint of a device according to one or more embodiments shown and described herein; and
FIG. 7 shows a side view of an embolisation device according to one or more embodiments shown and described herein.

### Detailed description

Throughout this disclosure the term `embolisation device' may refer to a device which may be permanently or semi-permanently implanted in a bodily lumen. Accordingly, the `embolisation device' may be configured to be disposed within the bodily lumen for a period of time, such as a number of days, or disposed in the lumen indefinitely. To this end, the `embolisation device' may be configured to be selectively detached from a delivery element so that it may be implanted in the bodily lumen in isolation.

Throughout this disclosure the term `bodily lumen' may refer to the inside space within a tubular structure of the human or animal body. The `bodily lumen' may be, for example, an artery or vein.

Throughout this disclosure the term `collapsed delivery configuration' of an element may refer to a configuration of the element which has a smaller radial extent than an expanded deployed configuration of the element.

Throughout this disclosure the term 'to anchor' may refer to partly or fully securing an element in a position.

Throughout this disclosure, the term 'stem' may refer to an elongate element which extends longitudinally along the length of the embolisation device to act as a backbone for the device.

Throughout this disclosure, the term "bristle segment" may refer to a group of bristles wherein the spacing between adjacent bristles is less than a predetermined distance. When two bristle segments are "spaced apart", the spacing between the bristle segments (i.e. the spacing between the most distal bristle of the first segment and the most proximal bristle of the second segment) is greater than the spacing between adjacent bristles within at least one of the bristle segments.

Bristles of an embolization device, when present, may be made of a flexible or resiliently deformable material such as stainless steel, Elgiloy or Nitinol. Other suitable materials may also be used, such as any suitable polymer or any other shape memory metal or metal alloy. The flow restrictor may be a thin film membrane made of a self-expanding material such as a polymer, stainless steel or Nitinol. The stem may be made of stainless steel or other suitable material and may comprise a twisted wire from which the bristles extend and on which the flow restrictor is mounted. The stem may alternatively comprise a hollow tube wherein the walls of the hollow tube hold the radially extending bristles in place. For example, the tube may be formed from a heat shrinkable material. Alternatively or additionally, the bristles may be held by the stem using other means such as adhesives.

The diameter of an individual flexible bristle may range from 0.036mm (0.0014 inches) to 0.053mm (0.0021 inches). For example, the diameter of an individual flexible bristle may be 0.0381mm (0.0015 inches), 0.0445mm (0.00175 inches) or 0.0508mm (0.002 inches). The average radial diameter of the radial profile formed by the expanded flexible bristles may range from 5mm to 30mm.

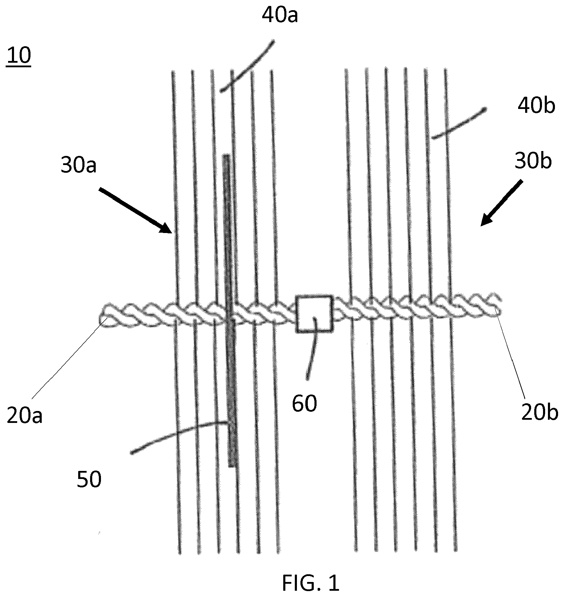

Fig. 1 shows an exemplary embolization device 10 in an unconstrained configuration. The embolization device comprises a plurality of flexible bristles 40a, 40b having deployed and collapsed configurations. The device comprises a series of segments wherein the bristles 40a of at least one segment 30a are configured to point distally when deployed in a bodily lumen, and the bristles 40b of at least one segment 30b are configured to point proximally when deployed in a bodily lumen. In some cases there is only a proximal segment 30a and distal segment 30b.

A proximally pointing segment is defined as a segment in which the bristles point proximally. The proximally pointing segment 30a may comprise a flow restrictor 50. The flow restrictor may be a cone open at the proximal end. The flow restrictor 50 comprises a hole (not shown) through which the stem 20a passes such that the flow restrictor 50 is mounted on the stem 20a. A distally pointing segment is defined as a segment in which the bristles point distally. The flow restrictor 50 may alternatively be present on the distally pointing segment and may be a cone open at the distal end.

At least one segment, in this case the proximal segment 30a, incorporates a flow restrictor 50. The flow restrictor 50 is configured to restrict bloodflow in the vessel when the device 10 is deployed.

In some examples a series of radiopaque markers divides the proximally pointing segment 30a and the distally pointing segment 30b, such as the radiopaque marker 60 shows in FIG. 1. It will be appreciated that the radiopaque marker 60 may alternatively be positioned proximally to the most proximal segment or distally to the most distal segment, or in between segments. The device may comprise one or more markers 60. There may be a proximal marker proximal to the proximal bristle segment 30a (not shown), a distal marker distal to the distal segment (not shown), and an intermediate marker 60. In other examples, the device does not comprise any markers.

In some examples the bristles 40a, 40b are formed on respective stems 20a, 20b. In the illustrated example the stems 20a, 20b comprise a twisted metal wire which securely holds the bristles in the twists of the wire. The stems 20a, 20b may alternatively comprise a hollow tube wherein the walls of the hollow tube hold the radially extending bristles in place. For example, the tube may be formed from a heat shrinkable material. Alternatively or additionally, the bristles may be held by the stem using other means such as adhesives.

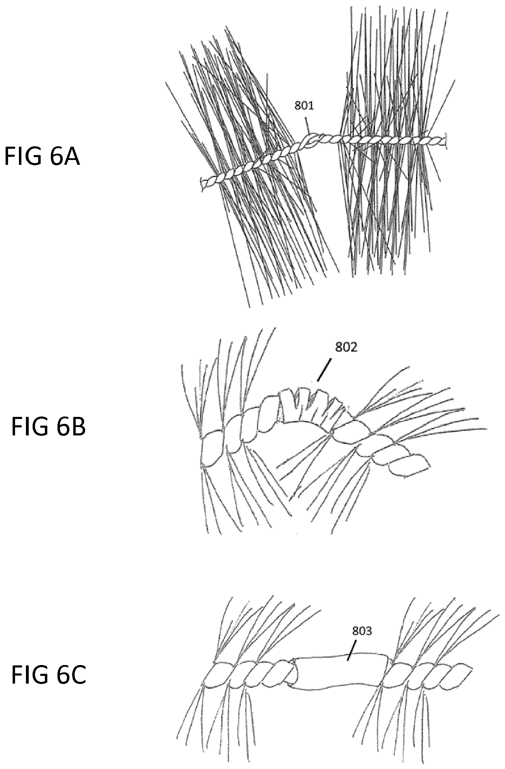

The stems 20a, 20b may be separately formed and joined by an articulating, flexible or non-articulating joint. For example, in the illustrated example the stems 20a, 20b are connected via the radiopaque marker 60 which is securely fastened to the stems 20a, 20b and which is made of an inflexible material. Alternatively, the marker 60 may merely be a radiopaque band situated at a location on one of the stems or the joint, and the stems 20a, 20b may be joined by an articulating joint 801 such as that shown in Fig. 6A, a flexible joint 802 such as the flexible tube 802 of Fig. 6B which comprises slots to accommodate bending of the tube, or an inflexible joint 803 such as that shown in Fig. 6C which may be a crimping connector. In other examples, the bristle segments may be formed on a single stem and the stem may be more or less flexible at the section joining the bristle segments.

In one case the embolization device comprises only a single proximal segment 30a and a single distal segment 30b. The proximal segment 30a and the distal segment 30b in one case are mounted on a single common stem. The stem 20a of the proximal segment 30a and the stem 20a of the distal segment 30b may form parts of the same continuous stem.

In the case where the device comprises more than two segments, the connection between the two most proximal segments may be more stiff than the distal connections. The distal connections may generally comprise a hinge.

In one example, a flow restrictor 50 comprising a flexible membrane is present in at least one of the segments. The membrane may comprise a disc of thin film material. The flexibility of the membrane means its orientation is controlled by the orientation of the adjacent bristles - in the illustrated example, if the adjacent bristles 40a are forced to point distally the membrane will adjust its configuration accordingly. Thus, if the membrane is deployed from a collapsed condition, such as from within a catheter, the bristles will cause it to open up to an expanded configuration. The membrane may also be placed proximal or distal to the segment.

Optionally, the membrane may comprise lines along which the membrane preferentially collapses when it is in the collapsed configuration, so that folding of the membrane in the collapsed configuration is controlled in a predictable manner.

In one case, the embolization device 10 comprises at least two segments. In one configuration the flow restrictor 50 is in the most proximal segment. This is shown in an unconstrained state schematically in Fig. 1. In the configuration shown the flow restrictor 50 is located within the proximal segment 30a with bristles 40a both proximal and distal to the flow restrictor 50. In some cases there may be a distal flow restrictor (not shown), and there may be any number of flow restrictors provided on the embolization device.

The flow restrictor 50 may have an outer dimension which is less than an outer dimension of the plurality of anchoring bristles. The flow restrictor may be connected to the stem. In some examples the flow restrictor may have a central hole that is an interference fit on the stem. The central hole in the flow restrictor is preferably smaller than the stem on which it is mounted. The central hole in the flow restrictor may have a diameter which is smaller than the diameter of the stem prior to assembly of the device.

The device 10 has a collapsed configuration to facilitate delivery through a catheter. By placing the flow restrictor 50 within the bristles 40a of the segment 20a, i.e. with bristles proximal and distal to it, it is protected from damage while the implant is being collapsed or pushed through a catheter. Furthermore, any friction between the catheter and the flow restrictor 50 is reduced.

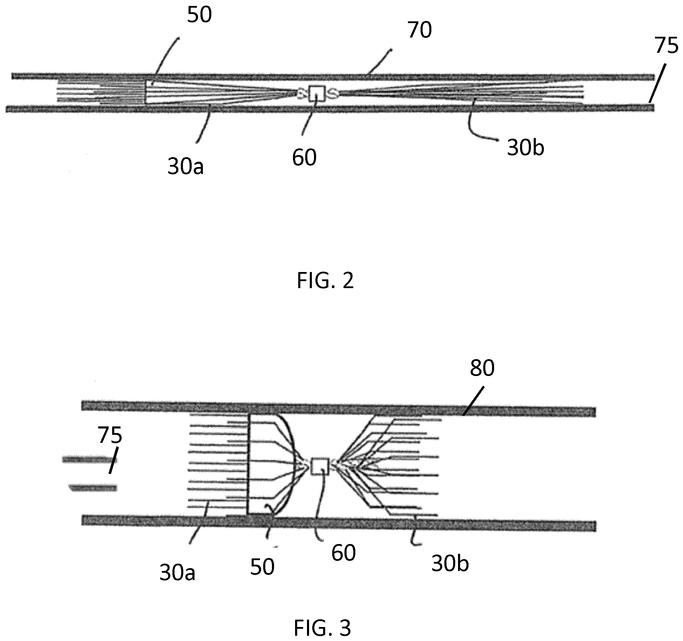

In one configuration, the implant is collapsed such that the bristles 40a of the most proximal segment 30a point proximally, while the bristles 40b of the distal segment 30a or segments point distally. Since the flow restrictor orientation is controlled by the orientation of the bristles, if the flow restrictor 50 is within the proximal segment, it will also point proximally. This is shown schematically in Fig. 2, with the device 10 shown in a collapsed delivery configuration within a delivery catheter 70.

A distal end 75 of the catheter 70 is positioned at a target location within a bodily lumen, and the embolization device 10 is delivered from the catheter 70 into the bodily lumen from the distal end. For example, a delivery element (not shown) may be used to push the embolization device 10 through the delivery catheter 70. The delivery element may be moved distally relative to the delivery catheter 70 to delivery the embolization device 10 from the distal end 75.

Fig. 3 shows the embolization device 10 in an expanded deployed configuration within a bodily lumen 80, after being delivered from the distal end 75 of the delivery catheter 70. The plurality of flexible bristles anchor the embolization device 10 to the bodily lumen 80.

For many embolization devices the bristles are assembled on the stem before the flow restrictor is mounted on the stem. In such instances, the bristles may be manipulated into a collapsed configuration and the flow restrictor passed over the collapsed bristles to reach the correct mounting location.

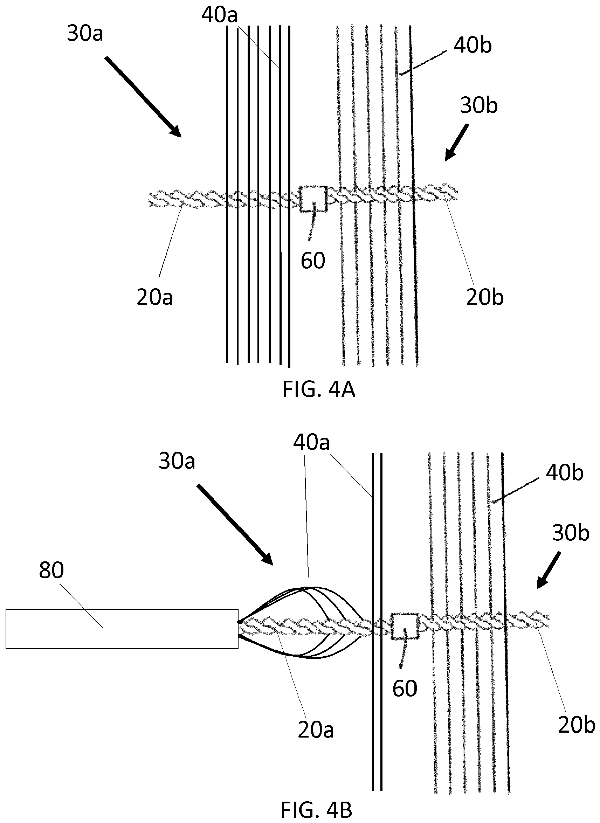

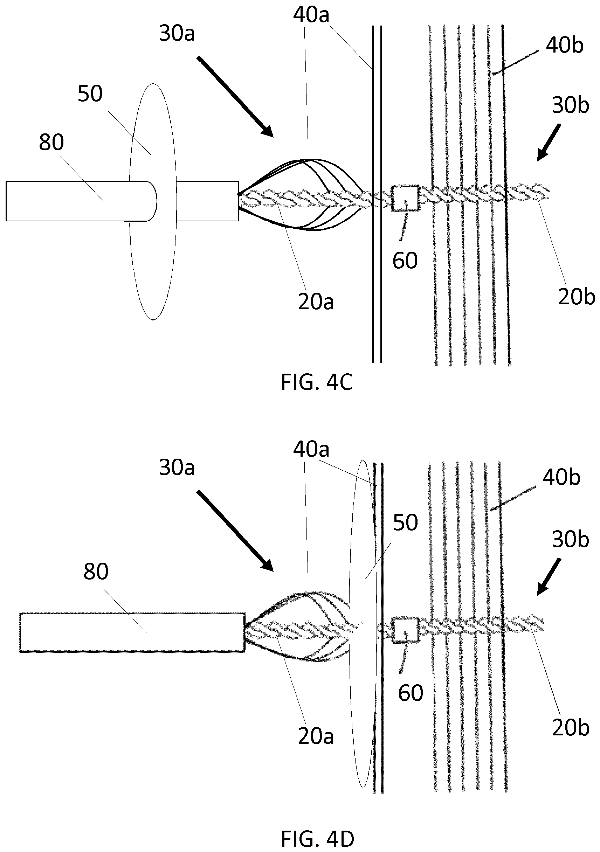

An exemplary process of mounting a flow restrictor onto a stem of an embolization device is now described with reference to Figs. 4A to 4D.

Fig. 4A shows the embolization device 10 before the flow restrictor 50 has been mounted on the stem 20b of the proximal bristle segment 30a.

Using a bristle manipulator tool 80, a plurality of bristles 40a of the proximal bristle segment 30a are manipulated into a collapsed configuration using a bristle manipulator tool 80, as illustrated in Fig. 4B. It is noted that the diameter of the bristle manipulator 80 is exaggerated for the purposes of clarity, whereas the actual diameter of the bristle manipulator may be closer to the diameter of the stem 20a. The skilled person will appreciate that an appropriate diameter for the bristle manipulating tool 80 can be selected such that the membrane is not damaged when mounted upon it.

For example, the bristle manipulator tool comprises a hollow tube into which the bristles are inserted such that they are held in the collapsed position. It will be appreciated by the skilled person that any suitable manipulator tool suitable for holding the bristles in a collapsed configuration can be used.

Next, as shown in Fig. 4C, the flow restrictor 50 is mounted on the bristle manipulator tool 80 such that the tool 80 passes through the hole of the flow restrictor 50.

Finally, the flow restrictor 50 is moved along the bristle manipulator tool 80 and over the collapsed bristles 40a onto the stem 20a of the proximal segment 30a. The bristle manipulator 80 may then be removed so that the collapsed bristles 40a return to their unconstrained configuration, such as that shown in Fig. 1.

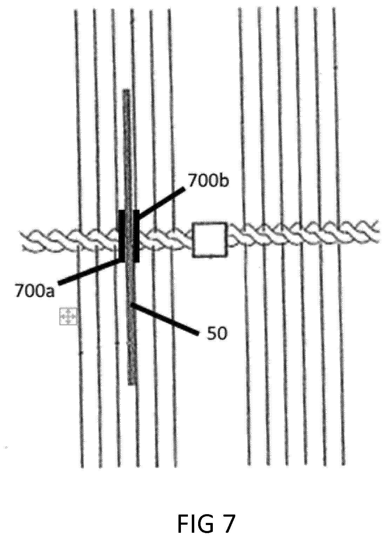

It is noted that the inner hole of the flow restrictor 50 could be dimensioned such that the flow restrictor 50 is securely mounted on the stem by an interference fit. Alternatively or additionally, after positioning the flow restrictor 50 on the stem, the flow restrictor 50 may be bonded to the stem by welding or adhesive. Alternatively or additionally, the flow restrictor may be mounted on the stem longitudinally between a first and second holding ring 700a and 700b, such as in the device shown in Fig. 7, the first and second holding rings attached to the stem by an interference fit, welding or adhesive.

The above process of mounting the flow restrictor imparts strain upon the hole of the flow restrictor 50. For example, the outer radius of the bristle manipulator tool 80 and the collapsed bristles is greater than the diameter of the stem 20a. At the same time, it is desirable for the hole of the flow restrictor 50 to be sufficiently small to create an interference fit between the flow restrictor 50 and the stem 20a (i.e. that the diameter of the hole in a relaxed state, when the stem does not pass through the hole, is smaller than the diameter of the stem). Accordingly, the hole of the flow restrictor 50 undergoes strain during the assembly process which makes the membrane of the flow restrictor 50 prone to damage and more specifically tearing.

Even for embolization devices not comprising bristles or for devices where the flow restrictor is mounted on the embolization device as the most proximal or distal component, there is still a risk of damage to the flow restrictor during assembly due to the manipulation of the flow restrictor whilst it is being mounted onto the stem. For example, the hole of the flow restrictor may still have a smaller diameter than the stem in a relaxed state (i.e. in an original state before the stem is inserted) and thus may still be prone to tearing during assembly.

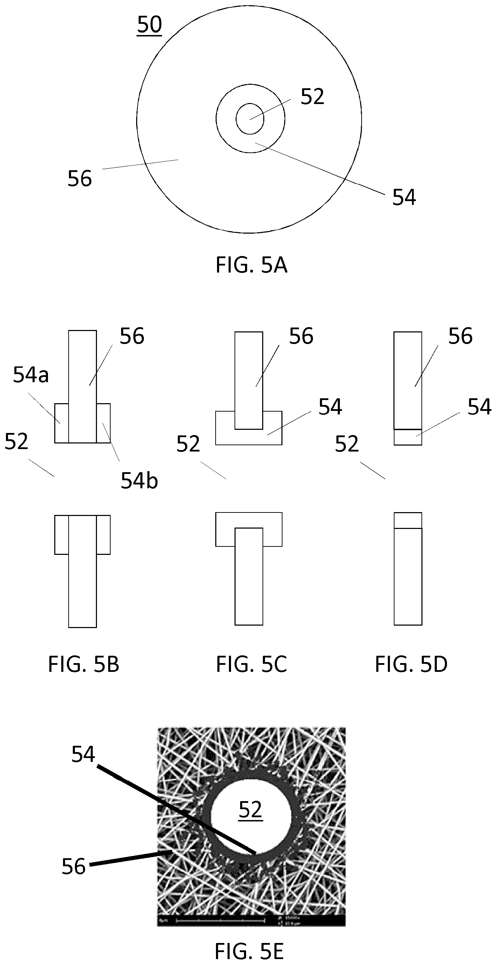

Fig. 5A shows an exemplary flow restrictor 50 for an embolization device according to the present disclosure (for example the embolization device shown in Figs. 1 to 3). The flow restrictor 50 comprises a membrane 56, a reinforcing section 54 and an inner hole 52 extending through the flow restrictor 50. The reinforcing section 54 has a first elasticity and the outer section 56 has a second elasticity different to the first elasticity. The reinforcing section 54 provides reinforcement to the flow restrictor 50 where strain is experienced during assembly (i.e. about the inner hole 52, as discussed above). In the illustrated example, the reinforcing section 54 entirely (i.e. completely) surrounds the inner hole 52 and defines the perimeter of the inner hole 52. In other examples, the reinforcing section 54 only partially surrounds the perimeter of the inner hole 52. For example, in some examples the flow restrictor 50 is prone to higher strain along certain portions of the perimeter of the inner hole 52 (for example during assembly of the embolization device) and the reinforcing section 54 may therefore only be placed in places of higher strain. In yet further examples, the reinforcing section 54 does not define the perimeter of the inner hole 52 but is placed adjacent to the perimeter of the inner hole 52.

In the illustrated example, the membrane 56 is annular in shape whereas in other examples the membrane 56 takes other shapes. For example, the membrane 56 may have an oval, triangular, square, star or any other shape. The membrane 56 may comprise slots extending radially inwards from the outer circumference to assist in preferable folding.

As explained in further detail in relation to Figs. 5B to 5D, the reinforcing section 54 and membrane 56 may abut each other, i.e. a dividing line may be drawn between them. In other examples according to the appended claims, the reinforcing section 54 and membrane 56 intersperse with one another. For example, the membrane 56 may be made of a fibrous material and the reinforcing section may be interspersed between the fibers of the membrane material. In some examples the reinforcing section 54 may also be made of a fibrous material.

The example illustrated in Fig. 5A shows the membrane defining the entire outermost perimeter of the flow restrictor, whereas in other examples the membrane only defines a part of the outermost perimeter.

Whilst in the illustrated example of Fig. 5A both the reinforcing section 54 and the membrane have an annular shape, in other examples they
The illustrated example in Fig. 5A shows the reinforcing section 54 as annular, whereas in other examples the reinforcing section may take a different shape such as oval, triangular, square, star or any other shape.

As described in further detail below, in some examples the elasticity of the reinforcing section 54 is greater than the elasticity of the membrane 56, which assists the inner hole in relaxing (i.e. returning) to its original diameter (to mount the flow restrictor 50 securely on the stem), whereas in other examples the elasticity of the reinforcing section 54 is less than the elasticity of the membrane 56, which provides rigidity to the inner hole 52 and reduces the risk of tearing of the membrane 56 during mounting on the stem.

Figs. 5B to 5E show cross sections of exemplary membranes according examples not falling within the scope of the appended claims.

In Fig. 5B, the reinforcing section 54 comprises a first annular portion 54a formed on a first planar side of the membrane 50, and a second annular portion 54b formed on a second planar side of the membrane 50. The inner hole 52 extends through the first and second portions 54a, 54b and the membrane 56, such that the inner hole 52 is defined by the first annular portion 54a, the membrane 56 and the second annular portion 54b.

Fig. 5C shows a cross section of another exemplary membrane according to the present disclosure. In Fig. 5C, similar to the example shown in Fig. 5B, the reinforcing section 54 comprises portions formed on either planar side of the membrane 50. However, the portions are joined such that the radial inner surface of the reinforcing section 54 completely defines the inner hole 52 along its length.

Fig. 5D shows a cross section of yet another exemplary membrane according to the present disclosure. In Fig. 5D, the reinforcing section 54 is formed such that it abuts the membrane 56 only radially (rather than on the planar surfaces of the membrane 56 as shown in Figs. 5B and 5C). As in the case of the example shown in Fig. 5C, the radial inner surface of the reinforcing section 54 completely defines the inner hole 52.

Figs. 5B to 5D show the reinforcing section 54 and the membrane 56 abutting one another. However, in other examples the reinforcing section 54 and membrane 56 may overlap each other, for example by intertwining or by the reinforcing section permeating the membrane 56. Fig. 5E illustrates one such example. In the illustrated example, the membrane 56 comprises a fibered matrix and the reinforcing section 54 surrounds the inner hole 52 and permeates the membrane 56 (i.e. extends between the fibers of the membrane 56).

The membrane 56 may comprise or consist of any polymer suitable for mechanically occluding a vessel whilst having a suitable lubricity for being delivered to the vessel through a delivery catheter, such as any polymers which may be formed of a thin film, for example ultra-high-molecular-weight polyethylene (UHMWPE), polytetrafluoroethylene, such as an expanded polytetrafluoroethylene (ePTFE) or an electrospun polytetrafluoroethylene, a polytetrafluoroethylene composite, silicone polycarbonate urethane or polycarbonate urethane. The membrane 56 may be a composite, for example comprising a plurality of laminates or otherwise joined materials. The membrane 56 may comprise or consist of a fibrous material. For example, the membrane 56 may comprise or consist of fibrous electrospun PTFE, UHMWPE or ePTFE.

The membrane 56 may be a thin film membrane having a thickness of between 4 to 100 microns. More specifically, the thickness may be between 4 to 35 microns, and even more specifically between 4 to 10 microns.

As previously stated, the elasticity of the reinforcing section 54 may be greater or less than the elasticity of the membrane 56.

In examples where the elasticity of the reinforcing section 54 is greater than the elasticity of the membrane, the reinforcing section 54 may comprise or consist of a thermoplastic material. The reinforcing section 54 may comprise or consist of a polyurethane such as silicone polycarbonate urethane or polycarbonate urethane. The material of the reinforcing section 54 may be in fibrous or non-fibrous form.

In examples where the elasticity of the reinforcing section 54 is less than the elasticity of the membrane, the reinforcing section 54 may comprise or consist of an adhesive such as cyanoacrylate.
in some embodiments, the diameter of the reinforcing section is less than 40% of the average diameter of the membrane. This ensures that the lubricity of the flow restrictor when being delivered through a delivery catheter is mainly determined by the surface properties of the membrane (i.e. the outermost parts of the flow restrictor which abut the inner surface of the delivery catheter - see Fig. 2, foe example), improving the deliverability of the flow restrictor.

The average diameter of the reinforcing section may be greater than 5% of the average diameter of the membrane. Diameters in this range have shown to provide a good amount of resistance to tearing of the flow restrictor at the inner hole.

How the flow restrictors disclosed herein may be formed is now discussed in further detail.

A method of manufacturing a flow restrictor for an embolization device comprises the steps of:
- providing a membrane for restricting flow in a bodily lumen, having a first elasticity;
- providing a reinforcing section having a second elasticity; and
- providing an inner hole extending through the flow restrictor, wherein the reinforcing section at least partially surrounds the inner hole.

The step of providing the membrane of the flow restrictor may comprise providing a layer of first material or materials. For example, a layer of first material may be provided by electrospinning, spin casting or spray casting, providing one ore more laminates or any other suitable method.

It is noted that the inner hole may be provided before or after the reinforcing section is provided.

The step of providing the reinforcing section may comprise adding a second material to the first material in and/or on a portion of the first material. This may occur before the inner hole is provided. The inner hole may then be formed through a combination of the first material and the second material.

In other examples, the inner hole may be formed in the layer of first material before the second material is added.

The first material may be a fibrous material. In some examples where the first material is a fibrous material, the reinforcing section may be provided by adding a second material to the fibrous first material when the second material is in a liquid phase. In other examples the second material may also be a fibrous material.

For example, the first material may be UHMWPE. The second material may be TPU, applied to the UHMWPE in a liquid phase such that the TPU is interspersed amongst the UHMWPE. Such a technique provides a flow restrictor having the mechanical properties for delivery through a catheter and occlusion within a vessel, whilst exhibiting high flexibility at the inner hole such that tearing of the flow restrictor at the hole is inhibited.

How an embolization device according to the present disclosure may be formed is now discussed in further detail.

A method of manufacturing an embolization device comprises the steps:
- providing a stem;
- providing a flow restrictor, the flow restrictor having an inner hole therein, the flow restrictor comprising: a membrane for restricting flow in the bodily lumen, having a first elasticity; and a reinforcing section having a second elasticity and at least partially surrounding the inner hole; and
- disposing the flow restrictor on the stem such that the stem passes through the inner hole.

Disposing the flow restrictor on the stem may comprise stretching the inner hole in at least one direction and thereafter passing the stem therethrough. Disposing the flow restrictor on the stem further comprises releasing the inner hole to form a frictional fit between the inner hole and the stem. The fit formed between the flow restrictor and the stem may lead to a fit called an interference fit.

In examples where the reinforcing section comprises a thermoplastic material, disposing the flow restrictor on the stem may comprise heating the thermoplastic material of the reinforcing section, for example to just below its transition phase, before passing the stem through the inner hole.

Table 1 below contains exemplary dimensions for the membrane and reinforcing section.

**Table 1**

| Membrane thickness | Membrane Diameter | Reinforcing section diameter |
|---|---|---|
| 4 - 35 microns | 6.5mm | 1-2mm |
| 4 - 35 microns | 9mm | 1-3.5mm |
| 4 - 35 microns | 16mm | 1-3.5mm |

The membranes disclosed herein have been described in relation to an embolization device with a core and a plurality of flexible bristles. It will be appreciated that the membrane may be mounted at any suitable location on the embolization device. Whilst disclosed in relation to devices comprising a plurality of flexible bristles, the membranes are also applicable to any type of embolization device.

All of the above are fully within the scope of the present disclosure, and are considered to form the basis for alternative embodiments in which one or more combinations of the above described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

## Claims

1. A flow restrictor (50) for an embolization device (10), the flow restrictor (50) having an inner hole (52) for receiving a stem (20a, 20b) of the embolization device (10) to mount the flow restrictor (50) on the embolization device (10),
wherein the flow restrictor (50) comprises:
a membrane (56) for restricting flow in the bodily lumen (80), having a first elasticity; and
a reinforcing section (54) having a second elasticity and at least partially surrounding the inner hole (52);
**characterised in that**
the reinforcing section (54) is at least partially interspersed with the membrane (56).

2. The flow restrictor (50) of claim 1, wherein the reinforcing section (54) entirely surrounds the perimeter of the inner hole (52), and preferably wherein the reinforcing section (54) defines the entire perimeter of the inner hole (52).

3. The flow restrictor (50) of claim 2, wherein the reinforcing section (54) is annular in shape, and wherein the inner radial surface of the annulus defines the inner hole (52).

4. The flow restrictor (50) of any preceding claim, wherein the membrane (56) is annular in shape.

5. The flow restrictor (50) of any preceding claim, wherein the membrane (56) defines at least a part of the outermost perimeter of the flow restrictor (50), and, optionally, wherein the membrane (56) defines the outermost perimeter of the flow restrictor (50).

6. The flow restrictor (50) of any preceding claim, wherein the average diameter of the reinforcing section (54) is less than 40% of the average diameter of the membrane (56), and/or wherein the average diameter of the reinforcing section (54) is greater than 5% of the average diameter of the membrane (56).

7. The flow restrictor (50) of any preceding claim, wherein the second elasticity is greater than the first elasticity, optionally wherein the reinforcing section (54) comprises or consists of a polyurethane such as silicone polycarbonate urethane or polycarbonate urethane.

8. The flow restrictor (50) of any one of claims 1 to 6, wherein the second elasticity is lower than the first elasticity.

9. The flow restrictor (50) of any preceding claim, wherein the reinforcing section (54) comprises or consists of a thermoplastic material and/or wherein the membrane (56) comprises or consists of:
ultra-high-molecular-weight polyethylene;
polytetrafluoroethylene, such as an expanded polytetrafluoroethylene or an electrospun polytetrafluoroethylene;
a polytetrafluoroethylene composite;
silicone polycarbonate urethane; or
polycarbonate urethane.

10. An embolization device (10) for promoting clot formation in a bodily lumen (80), the embolization device (10) adapted to have a collapsed delivery configuration and an expanded deployed configuration, the embolization device (10) comprising:
a stem (20a, 20b); and
a flow restrictor (50) according to any preceding claim and being disposed on the stem (20a, 20b) such that the stem (20a, 20b) passes through the inner hole (52).

11. A method of manufacturing a flow restrictor (50) for an embolization device (10), the method comprising:
providing a membrane (56) for restricting flow in a bodily lumen (80), having a first elasticity;
providing a reinforcing section (54) having a second elasticity; and
providing an inner hole (52) extending through the flow restrictor (50),
wherein the reinforcing section (54) at least partially surrounds the inner hole (52), **characterised in that**
the reinforcing section (54) is at least partially interspersed with the membrane (56).

12. A method of manufacturing an embolization device (10), the method comprising the steps of claim 11 and further comprising:
providing a stem (20a, 20b) of the embolization device (10); and
disposing the flow restrictor (50) on the stem (20a, 20b) such that the stem (20a, 20b) passes through the inner hole (52).

13. The method of claim 12, wherein disposing the flow restrictor (50) on the stem (20a, 20b) comprises stretching the inner hole (52) in at least one direction, thereafter passing the stem (20a, 20b) therethrough and thereafter releasing the inner hole (52) to form a frictional fit between the inner hole (52) and the stem (20a, 20b).

14. The method of any one of claims 12 or 13, wherein:
disposing the flow restrictor (50) on the stem (20a, 20b) comprises forming an interference fit between the flow restrictor (50) and the stem (20a, 20b); and/or
disposing the flow restrictor (50) on the stem (20a, 20b) comprises bonding the flow restrictor (50) to the stem (20a, 20b) by welding or adhesive; and/or
disposing the flow restrictor (50) on the stem (20a, 20b) comprises disposing the flow restrictor (50) longitudinally between a first and second holding ring (700a, 700b), the first and second holding rings (700a, 700b) attached to the stem (20a, 20b) by an interference fit, welding or adhesive.

15. The method of any one of claims 12 to 14, wherein the reinforcing section (54) comprises or consists of a thermoplastic material, optionally wherein the disposing the flow restrictor (50) on the stem (20a, 20b) comprises heating the thermoplastic material of the reinforcing section (54) before passing the stem (20a, 20b) through the inner hole (52).

## Patentansprüche

1. Durchflussbegrenzer (50) für eine Embolisierungsvorrichtung (10), wobei der Durchflussbegrenzer (50) ein inneres Loch (52) zur Aufnahme eines Schafts (20a, 20b) der Embolisierungsvorrichtung (10) aufweist, um den Durchflussbegrenzer (50) an der Embolisierungsvorrichtung (10) zu befestigen,
wobei der Durchflussbegrenzer (50) umfasst:
eine Membran (56) zur Begrenzung des Durchflusses im Körperlumen (80), die eine erste Elastizität aufweist; und
einen Verstärkungsabschnitt (54), der eine zweite Elastizität aufweist und das innere Loch (52) zumindest teilweise umgibt;
**dadurch gekennzeichnet, dass**
der Verstärkungsabschnitt (54) zumindest teilweise von der Membran (56) durchsetzt ist.

2. Durchflussbegrenzer (50) nach Anspruch 1, wobei der Verstärkungsabschnitt (54) den Umfang des inneren Lochs (52) vollständig umgibt, und vorzugsweise wobei der Verstärkungsabschnitt (54) den gesamten Umfang des inneren Lochs (52) definiert.

3. Durchflussbegrenzer (50) nach Anspruch 2, wobei der Verstärkungsabschnitt (54) ringförmig ist, und wobei die innere radiale Oberfläche des Ringes das innere Loch (52) definiert.

4. Durchflussbegrenzer (50) nach einem vorstehenden Anspruch, wobei die Membran (56) ringförmig ist.

5. Durchflussbegrenzer (50) nach einem vorstehenden Anspruch, wobei die Membran (56) zumindest einen Teil des äußersten Umfangs des Durchflussbegrenzers (50) definiert, und, optional, wobei die Membran (56) den äußersten Umfang des Durchflussbegrenzers (50) definiert.

6. Durchflussbegrenzer (50) nach einem vorstehenden Anspruch, wobei der durchschnittliche Durchmesser des Verstärkungsabschnitts (54) weniger als 40 % des durchschnittlichen Durchmessers der Membran (56) beträgt, und/oder wobei der durchschnittliche Durchmesser des Verstärkungsabschnitts (54) mehr als 5 % des durchschnittlichen Durchmessers der Membran (56) beträgt.

7. Durchflussbegrenzer (50) nach einem vorstehenden Anspruch, wobei die zweite Elastizität größer ist als die erste Elastizität, wobei optional der Verstärkungsabschnitt (54) ein Polyurethan wie Silikon-Polycarbonat-Urethan oder Polycarbonat-Urethan umfasst oder daraus besteht.

8. Durchflussbegrenzer (50) nach einem der Ansprüche 1 bis 6, wobei die zweite Elastizität geringer ist als die erste Elastizität.

9. Durchflussbegrenzer (50) nach einem vorstehenden Anspruch, wobei der Verstärkungsabschnitt (54) ein thermoplastisches Material umfasst oder aus einem solchen besteht und/oder wobei die Membran (56) umfasst oder besteht aus:
ultrahochmolekulares Polyethylen;
Polytetrafluorethylen, wie z. B. ein expandiertes Polytetrafluorethylen oder ein elektrogesponnenes Polytetrafluorethylen;
ein Polytetrafluorethylen-Verbundstoff;
Silikon-Polycarbonat-Urethan; oder
Polycarbonat-Urethan.

10. Embolisierungsvorrichtung (10) zur Förderung der Gerinnselbildung in einem Körperlumen (80), wobei die Embolisierungsvorrichtung (10) angepasst ist, um eine kollabierte Zuführungskonfiguration und eine erweiterte entfaltete Konfiguration aufzuweisen, wobei die Embolisierungsvorrichtung (10) umfasst:
einen Schaft (20a, 20b); und
einen Durchflussbegrenzer (50) nach einem vorstehenden Anspruch, der auf dem Schaft (20a, 20b) so angeordnet ist, dass der Schaft (20a, 20b) durch das innere Loch (52) hindurchgeht.

11. Verfahren zur Herstellung eines Durchflussbegrenzers (50) für eine Embolisierungsvorrichtung (10), wobei das Verfahren umfasst:
Bereitstellen einer Membran (56) zur Begrenzung des Durchflusses in einem Körperlumen (80), die eine erste Elastizität aufweist;
Bereitstellen eines Verstärkungsabschnitts (54), der eine zweite Elastizität aufweist; und
Bereitstellen eines inneren Loches (52), das sich durch den
Durchflussbegrenzer (50) erstreckt,
wobei der Verstärkungsabschnitt (54) das innere Loch (52) zumindest teilweise umgibt, **dadurch gekennzeichnet, dass**
der Verstärkungsabschnitt (54) zumindest teilweise von der Membran (56) durchsetzt ist.

12. Verfahren zur Herstellung einer Embolisierungsvorrichtung (10), wobei das Verfahren die Schritte nach Anspruch 11 umfasst und weiter umfasst:
Bereitstellen eines Schafts (20a, 20b) der Embolisierungsvorrichtung (10); und
Anordnen des Durchflussbegrenzers (50) auf dem Schaft (20a, 20b) so dass der Schaft (20a, 20b) durch das innere Loch (52) hindurchgeht.

13. Verfahren nach Anspruch 12, wobei das Anordnen des Durchflussbegrenzers (50) auf dem Schaft (20a, 20b) das Dehnen des inneren Lochs (52) in mindestens einer Richtung, das anschließende Hindurchführen des Schafts (20a, 20b) und das anschließende Freigeben des inneren Lochs (52) umfasst, um einen Reibschluss zwischen dem inneren Loch (52) und dem Schaft (20a, 20b) herzustellen.

14. Verfahren nach einem der Ansprüche 12 oder 13, wobei:
das Anordnen des Durchflussbegrenzers (50) auf dem Schaft (20a, 20b) das Bilden einer Presspassung zwischen dem Durchflussbegrenzer (50) und dem Schaft (20a, 20b) umfasst; und/oder
das Anordnen des Durchflussbegrenzers (50) auf dem Schaft (20a, 20b) das Verbinden des Durchflussbegrenzers (50) mit dem Schaft (20a, 20b) durch Schweißen oder Kleben umfasst; und/oder
das Anordnen des Durchflussbegrenzers (50) auf dem Schaft (20a, 20b) das Anordnen des Durchflussbegrenzers (50) in Längsrichtung zwischen einem ersten und einem zweiten Haltering (700a, 700b) umfasst, wobei der erste und der zweite Haltering (700a, 700b) durch eine Presspassung, durch Schweißen oder durch Kleben an dem Schaft (20a, 20b) befestigt sind.

15. Verfahren nach einem der Ansprüche 12 bis 14, wobei der Verstärkungsabschnitt (54) ein thermoplastisches Material umfasst oder aus einem solchen besteht, wobei optional das Anordnen des Durchflussbegrenzers (50) auf dem Schaft (20a, 20b) das Erwärmen des thermoplastischen Materials des Verstärkungsabschnitts (54) umfasst, bevor der Schaft (20a, 20b) durch das innere Loch (52) geführt wird.

## Revendications

1. Limiteur de débit (50) pour un dispositif d'embolisation (10), le limiteur de débit (50) présentant un orifice interne (52) pour recevoir une tige (20a, 20b) du dispositif d'embolisation (10) de manière à monter le limiteur de débit (50) sur le dispositif d'embolisation (10),
dans lequel le limiteur de débit (50) comprend :
une membrane (56) pour limiter le débit dans une lumière corporelle (80), présentant une première élasticité ; et
une section de renfort (54) présentant une deuxième élasticité et entourant au moins partiellement l'orifice intérieur (52) ;
**caractérisé en ce que**
la section de renfort (54) est au moins partiellement entrecoupée par la membrane (56).

2. Limiteur de débit (50) selon la revendication 1, dans lequel la section de renfort (54) entoure entièrement le périmètre de l'orifice interne (52), et préférablement dans lequel la section de renfort (54) définit le périmètre entier de l'orifice interne (52).

3. Limiteur de débit (50) selon la revendication 2, dans lequel la section de renfort (54) est de forme annulaire, et dans lequel la surface radiale intérieure de l'anneau définit l'orifice intérieur (52).

4. Limiteur de débit (50) selon l'une quelconque des revendications précédentes, dans lequel la membrane (56) est de forme annulaire.

5. Limiteur de débit (50) selon l'une quelconque des revendications précédentes, dans lequel la membrane (56) définit au moins une partie du périmètre le plus extérieur du limiteur de débit (50), et, facultativement, dans lequel la membrane (56) définit le périmètre le plus extérieur du limiteur de débit (50).

6. Limiteur de débit (50) selon l'une quelconque des revendications précédentes, dans lequel le diamètre moyen de la section de renfort (54) est inférieur à 40 % du diamètre moyen de la membrane (56), et/ou dans lequel le diamètre moyen de la section de renfort (54) est supérieur à 5 % du diamètre moyen de la membrane (56).

7. Limiteur de débit (50) selon l'une quelconque des revendications précédentes, dans lequel la deuxième élasticité est plus grande que la première élasticité, facultativement dans lequel la section de renfort (54) comprend ou se compose d'un polyuréthane tel que du polycarbonate uréthane de silicone ou du polycarbonate uréthane.

8. Limiteur de débit (50) selon l'une quelconque des revendications 1 à 6, dans lequel la deuxième élasticité est plus faible que la première élasticité.

9. Limiteur de débit (50) selon l'une quelconque des revendications précédentes, dans lequel la section de renfort (54) comprend ou se compose d'un matériau thermoplastique et/ou dans lequel la membrane (56) comprend ou se compose de :
polyéthylène de poids moléculaire ultra-élevé ;
polytétrafluoroéthylène, tel qu'un polytétrafluoroéthylène expansé ou un polytétrafluoroéthylène électrofilé ;
un composite de polytétrafluoroéthylène ;
polycarbonate uréthane de silicone ; ou
polycarbonate uréthane.

10. Dispositif d'embolisation (10) pour favoriser la formation de caillot dans une lumière corporelle (80), le dispositif d'embolisation (10) étant adapté pour présenter une configuration d'administration repliée et une configuration déployée étendue, le dispositif d'embolisation (10) comprenant :
une tige (20a, 20b) ; et
un limiteur de débit (50) selon une quelconque revendication précédente et disposé sur la tige (20a, 20b) de sorte que la tige (20a, 20b) passe à travers l'orifice interne (52).

11. Procédé de fabrication d'un limiteur de débit (50) pour un dispositif d'embolisation (10), le procédé comprenant :
une fourniture d'une membrane (56) pour limiter le débit dans une lumière corporelle (80), présentant une première élasticité ;
une fourniture d'une section de renfort (54) présentant une deuxième élasticité ; et
une fourniture d'un orifice interne (52) s'étendant à travers le limiteur de débit (50),
dans lequel la section de renfort (54) entoure au moins partiellement l'orifice intérieur (52), **caractérisé en ce que** la section de renfort (54) est au moins partiellement entrecoupée par la membrane (56).

12. Procédé de fabrication d'un dispositif d'embolisation (10), le procédé comprenant les étapes selon la revendication 11 et comprenant en outre :
une fourniture d'une tige (20a, 20b) du dispositif d'embolisation (10) ; et
une disposition du limiteur de débit (50) sur la tige (20a, 20b) de sorte que la tige (20a, 20b) passe à travers l'orifice intérieur (52).

13. Procédé selon la revendication 12, dans lequel la disposition du limiteur de débit (50) sur la tige (20a, 20b) comprend un étirement de l'orifice intérieur (52) dans au moins une direction, puis une action consistant à faire passer la tige (20a, 20b) à travers ce dernier, et enfin, à relâcher l'orifice intérieur (52) de manière à former un ajustement par frottement entre l'orifice intérieur (52) et la tige (20a, 20b).

14. Procédé selon l'une quelconque des revendications 12 ou 13, dans lequel :
la disposition du limiteur de débit (50) sur la tige (20a, 20b) comprend une formation d'un ajustement avec serrage entre le limiteur de débit (50) et la tige (20a, 20b) ; et/ou la disposition du limiteur de débit (50) sur la tige (20a, 20b) comprend une mise en liaison du limiteur de débit (50) avec la tige (20a, 20b) par un soudage ou un adhésif ; et/ou
la disposition du limiteur de débit (50) sur la tige (20a, 20b) comprend une disposition du limiteur de débit (50) longitudinalement entre des première et deuxième bagues de retenue (700a, 700b), les première et deuxième bagues de retenue (700a, 700b) étant fixées à la tige (20a, 20b) par un ajustement avec serrage, un soudage ou un adhésif.

15. Procédé selon l'une quelconque des revendications 12 à 14, dans lequel la section de renfort (54) comprend ou se compose d'un matériau thermoplastique, facultativement dans lequel la disposition du limiteur de débit (50) sur la tige (20a, 20b) comprend un chauffage du matériau thermoplastique de la section de renfort (54) avant de faire passer la tige (20a, 20b) à travers l'orifice interne (52).
